# EUROPEAN PATENT SPECIFICATION

(11) **EP 2 621 437 B1**
(45) Date of publication and mention of the grant of the patent: **14.12.2016**
(21) Application number: 11828473.6
(22) Date of filing: 30.09.2011
(51) Int. Cl.: A61F 13/49, A61F 13/496, A61F 13/53

(54) **PANTS-TYPE DIAPER**
HOSENÄHNLICHE WINDEL
COUCHE DE TYPE CULOTTE

(30) Priority: 30.09.2010 JP 2010222213
(43) Date of publication of application: 07.08.2013
(73) Proprietor: Unicharm Corporation, Ehime 799-0111 (JP)
(72) Inventor: MISHIMA, Yoshitaka, Kanonji-shi Kagawa 769-1602 (JP); FUJIMOTO, Kazuya, Kanonji-shi Kagawa 769-1602 (JP)
(74) Representative: Peter, Julian
(86) International application number: PCT/JP2011/005557
(87) International publication number: WO 2012/042908

(56) References cited:
- EP-A2- 1 210 926
- JP-A- 2003 180 744
- JP-A- 2004 016 648
- JP-A- 2007 267 763
- JP-A- 2007 267 763
- JP-A- 2008 012 198
- JP-A- 2008 521 481
- US-A1- 2004 078 017
- US-A1- 2007 233 029

## Description

### Technical Field

The present disclosure relates to a pants-type diaper.

### Background Art

Conventionally, in a pants- type diaper, it is known that there is used a sandglass-type absorber which is small in width at a portion disposed in an inner-leg region of the pants-type diaper (for example, PTL 1).

US 2004/078017 A1 discloses a diaper with a core having grooves as folding lines, and furthermore provided with a plurality of compression lines.

### Citation List

### Patent Literature

[PTL 1] Japanese Unexamined Patent Application Publication No. 4-503168

### Summary of Invention

### Technical Problem

However, the Applicant found the following problem associated with the pants-type diaper described above.

In such a pants-type diaper, since an absorber disposed in a crotch region in which urine is excreted by a wearer is smaller in width, there might be a problem in that a possibility of a leakage of urine is high.

Therefore, in order to solve such a problem, in such a pants-type diaper, it has been considered to configure the diaper so as to increase the width of the absorber that is disposed in the inner-leg region of the pants-type diaper, thereby increasing a surface area of such an absorber.

However, according to such a structure, there has been a problem that a wearer has a sense of discomfort at the time of wearing the pants-type diaper.

Accordingly, the present invention has been made in view of the problem described above, and it is an object of the present invention to provide a pants-type diaper which is capable of reducing a sense of discomfort at the time of wearing while avoiding a leakage of urine.

### Solution to Problem

A diaper as defined in claim 1.

### Advantageous Effect(s) of Invention

As has been described above, according to the present invention, there can be provided a pants-type diaper which is capable of reducing a sense of discomfort at the time of wearing while avoiding a leakage of urine.

### Brief Description of Drawings

[fig.1]Fig. 1 is an external view seen from a rear of a pants-type diaper according to a first embodiment of the present invention.
[fig.2]Fig. 2 is an exploded view of the pants-type diaper according to the first embodiment of the present invention.
[fig.3]Fig. 3 is a plan view of an absorber of the pants-type diaper according to the first embodiment of the present invention.
[fig.4]Fig. 4 is a sectional view taken along the line X-X' in the plan view of the absorber of the pants-type diaper according to the present invention.
[fig.5]Fig. 5 is a view showing one example of dimensions of a compression line and a folding line in the absorber of the pants-type diaper according to the first embodiment of the present invention.
[fig.6]Fig. 6 is a plan view of the absorber of the pants-type diaper according to the first embodiment of the present invention.
[fig.7]Fig. 7 is a view for explaining a method of manufacturing the absorber of the pants-type diaper according to the first embodiment of the present invention.
[fig.8]Fig. 8 is a view for explaining a method of manufacturing the absorber of the pants-type diaper according to the first embodiment of the present invention.
[fig.9]Fig. 9 is a plan view of an absorber of a pants-type diaper according to an exemplary modification of the present invention.
[fig.10]Fig. 10 is a plan view of the absorber of the pants-type diaper according to the exemplary modification of the present invention.

### Description of Embodiments

### (First Embodiment of the Present Invention)

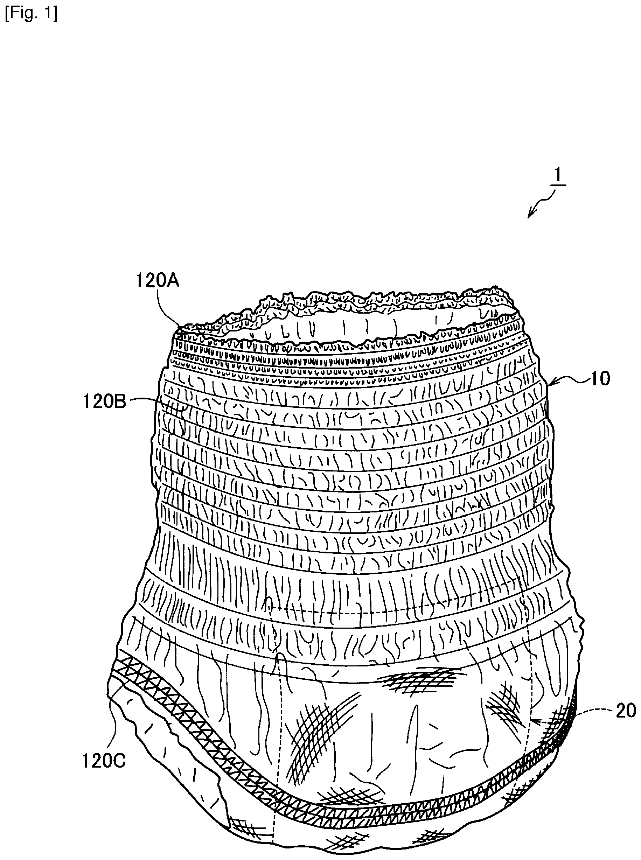

A pants-type diaper 1 according to a first embodiment of the present invention will be described with reference to Fig. 1 to Fig. 8.

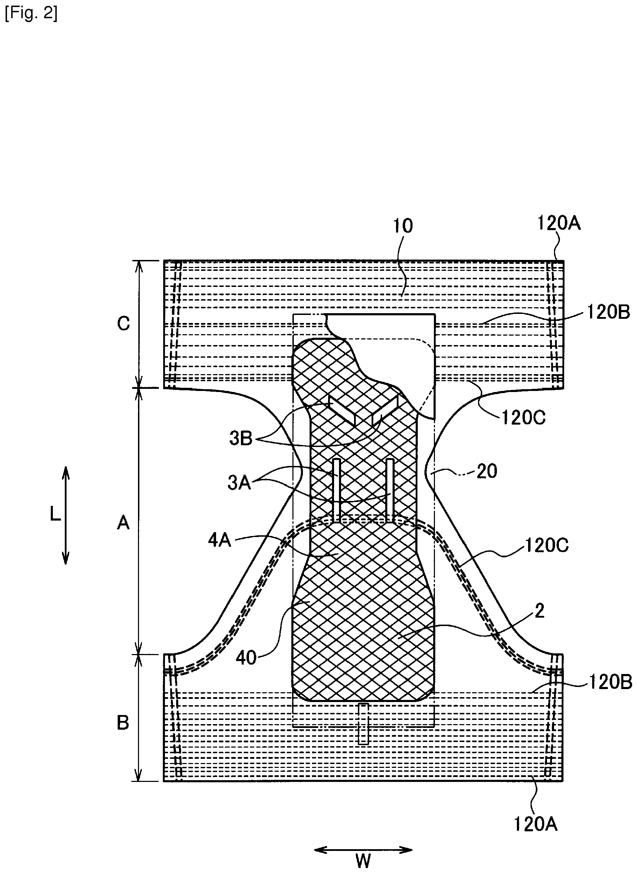

Fig. 1 shows an external view seen from a rear side of the pants-type diaper 1 according to the embodiment, and Fig. 2 shows an exploded view of the pants-type diaper 1 according to the first embodiment of the present invention.

As shown in Fig. 1 and Fig. 2, the pants-type diaper 1 according to the embodiment has an outer unit (chassis) 10 and an inner unit (absorber main body) 20.

As shown in Fig. 2, the outer unit 10 has an inner-leg region A, a front waistline region B, and a rear waistline region C.

Herein, the inner unit 20, as shown in Fig. 2, is provided from the inner-leg region A across the front waistline region B and the rear waistline region C at a skin contact surface side of the pants-type diaper 1 of the outer unit 10. As shown in Fig. 2, in the front waistline region B and the rear waistline region C, the inner unit 20 is provided in a center region in a widthwise direction W.

In addition, in the pants-type diaper 1 according to the embodiment, both side edge parts of the front waistline region B and both side edge parts of the rear waistline region C are configured so as to be bonded with each other.

Herein, at the outer unit 10, a waistline gather (waist gather) elastic member 120A, a waistline gather (fit gather) elastic member 120B, or a leg-line gather (leg gather) elastic member 120C are retained in an expanded state.

In addition, the inner unit 20 has a topsheet (not shown) which is a liquid permeable sheet; a backsheet (not shown) which is a liquid impermeable sheet; and an absorber 2 which is disposed between the topsheet and the backsheet.

In addition, at the inner unit 20, a leakage preventing wall (solid gather) having an elastic member which is extensive in a longitudinal direction L is provided at each side part in the longitudinal direction of the absorber 2.

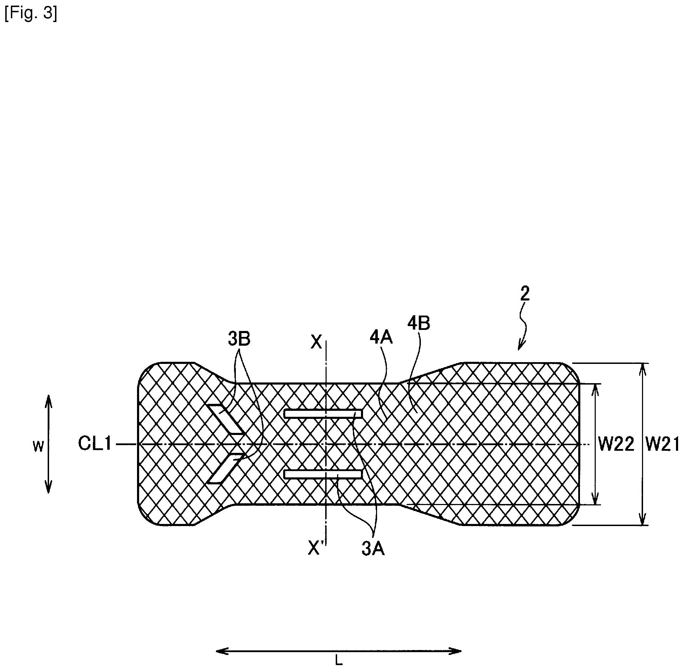

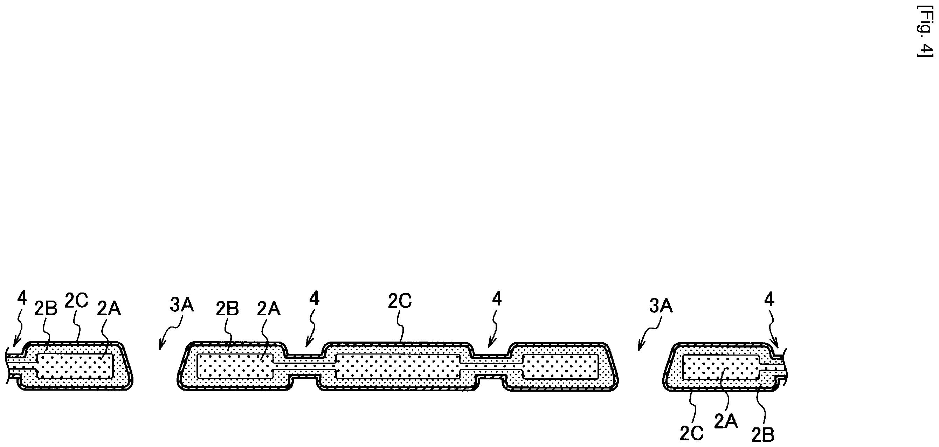

Fig. 3 shows a plan view of the absorber 2 when it is seen from a skin contact surface side of the pants-type diaper 1, and Fig. 4 shows a sectional view taken along the line X-X' in the plan view of such an absorber 2.

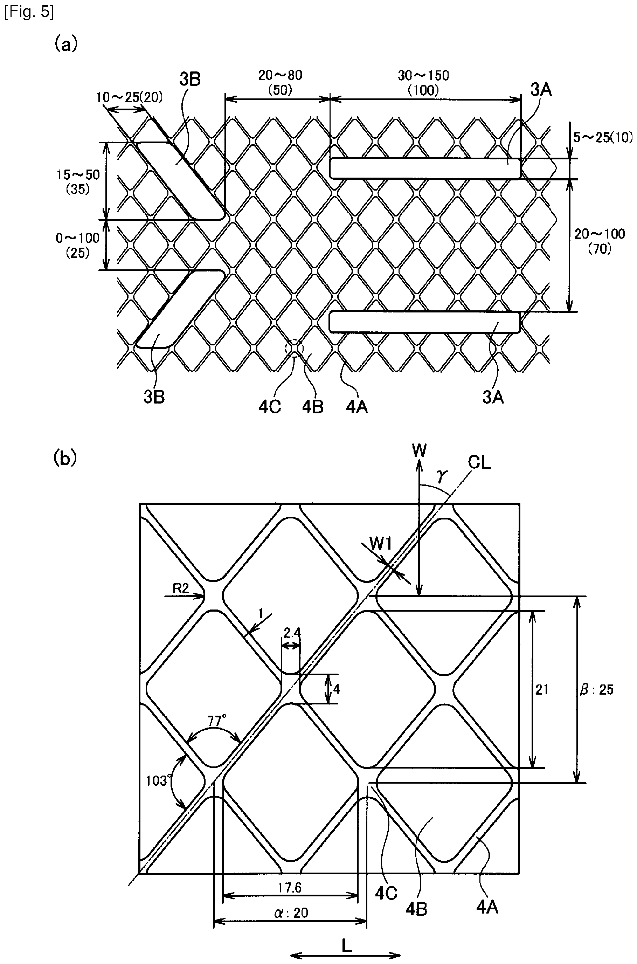

In addition, Fig. 5 (a) and Fig. 5 (b) each show one example of size of each part in a case where folding lines 3A/3B (to be described later) are formed by a slit, and a region 4B that is surrounded by compression lines 4A which are adjacent to each other is formed in the shape of a rhombus.

As shown in Fig. 4, the absorber 2 has a core made of an intermediate layer 2A and a pulp layer 2B in which pulp and SAP (super absorptive polymer) are mixed with each other, and is configured so that both faces of such a core are sandwiched by means of a covering sheet 2C.

It is desirable that: a basis weight of pulp is within a range from 100 g/m² to 500 g/m² (for example, 151 g/m²); and a basis weight of SAP is within a range from 0 g/m² to 300 g/m² (for example, 106 g/m²). If the weight of pulp is lower than the lower limit of 100 g/m², the absorber becomes too thin, and even with embossing it is not possible to achieve the targeted rigidity of the embossed area. Conversely, the upper limit of 500 g/m² makes the absorber too thick and the rigidity of the embossed area becomes high and makes the absorber rigid. The lower limit of the weight of SAP is set at 0 since embossing can be also effective in an absorber only made of pulp. As with the upper limit of the weight of pulp, the upper limit of the weight of SAP is set at 300 g/m² since any value above the limit makes the absorber rigid.

In addition, it is preferable that a SAP rate which is a ratio between the content of pulp and the content of SAP is within a range from 0% to 60% by mass (for example, 41% by mass). The lower limit of the weight of SAP rate is set at 0% since embossing can be also effective in an absorber only made of pulp. As with the upper limit of the weight of pulp, the upper limit of the weight of SAP rate is set at 60% since any value above the limit makes the absorber rigid.

Further, the absorber 2 may be rectangular in shape or may be of sandglass type. A sandglass-shaped absorber 2 is employed in the pants-type diaper 1 according to the embodiment.

As shown in Fig. 3, in the case of the absorber 2 of sandglass type, it is desirable that a ratio between a dimension W21 of the widest portion in the absorber 2 and a dimension W22 of the narrower portion in the absorber 2 is within a range from 5 : 3 to 1 : 1 (for example, 4 : 3). The lower rate of 5:3 is set since, in defining a wide absorber, it is the lower limit value at which the wide absorber becomes wider than current rehabilitation pants (the width of absorber is 2:1). If the ratio becomes any less than 5:3, the absorber cannot be regarded as a wide absorber. Therefore, the lower limit is set at 5:3.

On the other hand, in the case of the absorber 2 formed in a rectangular shape, a ratio between longitudinal and transverse lengths is within a range from 1 : 1 to 1 : 3 (for example, 10 : 27). The range for rate between longitudinal and transverse lengths is set on the grounds that since the rate is 1:1 and the shape is of a square, if the width of the absorber is any larger, it does not function as an absorber of pants-type diaper (to be specific, leakage of urine to wearer's back or belly frequently occurs), and also that even if the longitudinal length is made any longer than 1:3, it is not possible to effectively use the absorber from edge to edge.

As shown in Fig. 3, two folding lines 3A, which are extensive in the longitudinal direction L and are spaced in a widthwise direction W, are formed at the absorber 2. Herein, as shown in Fig. 3, the two folding lines 3A may be disposed to be spaced transversely symmetrically with respect to a centerline CL1 taken along the longitudinal direction L.

Such folding lines 3A are formed by slits. Herein, the slit, as shown in Fig. 4, shows a region in which pulp and SAP are not laminated.

When the folding lines 3A are formed by slits, since a rigidity difference between a region in which the folding lines 3A are formed and a peripheral region of folding lines 3A is increased more, a side part of the absorber 2 easily rises.

As shown in Fig. 4, an intermediate layer 2A in which pulp and SAP are mixed with each other does not exist on a wall face of the folding lines 3A (slits 3A), and a pulp layer 2B is configured so as to exist. That is, the intermediate layer 2A is configured so as to be disposed inside of the pulp layer 2B.

According to such a structure, a compression unit (emboss unit) is formed at the pulp layer 2B more easily than at the intermediate layer 2A. In addition, since a portion where a compression unit is formed in the pulp layer 2B existing on the wall face of slits 3A is sealed, SAP is hardly produced, the slits 3A are hardly deformed, and bendability of the absorber 2A is sustained.

In addition, at least such two folding lines 3A are configured so as to be formed in a region of the absorber 2 that is disposed in a crotch region of the pants-type diaper 1.

For example, as shown in Fig. 5 (a), it is desirable that a spaced distance between the slits 3A is within a range from 20 mm to 100 mm (for example, 70 mm); it is desirable that a length of the slit 3A is within a range from 30 mm to 150 mm (for example, 95 mm); and it is desirable that a width of a groove of the slit 3A is within a range from 5 mm to 25 mm (for example, 1mm).

In addition, the slit 3A may be curved toward a front end part or a rear end part in the longitudinal direction L of the absorber 2, or alternatively, may be curved toward both edge parts in the widthwise direction W of the absorber 2. Herein, the curving slit 3A can act uniformly on a force which is applied in a wearer's femoral direction.

In addition, as shown in Fig. 3, for example, folding lines 3B (slits 3B) may be additionally formed to folding lines 3A.

Such slits 3B may be disposed so as to be inclined at a predetermined angle with respect to each of the slits 3A. For example, an inclination angle of the slit 3B with respect to the slit 3A may be identical to an inclination angle of the compression line 4A (to be described later) with respect to the slit 3A.

According to such a structure, the absorber 2 is easily movable with respect to movement of the wearer's inner-legs and legs, and a sense of discomfort at the time of walking with respect to the wearer can be reduced. Specifically, the slit 3A corresponds to movability in the widthwise direction W of the wearer's inner legs, and the slit 3B corresponds to movability in the longitudinal direction L (forward/backward direction) of the wearer's legs.

In addition, folding lines (slits) may be formed on the centerline CL1 in the longitudinal direction L (not shown).

Further, as shown in Fig. 3, a plurality of compression lines 4A which are extensive in the widthwise direction W and are spaced in the longitudinal direction L are formed at the absorber 2. Herein, such compression lines 4A are configured so as to be formed in a predetermined region of the absorber 2 in which the folding lines 3A are formed. Such a predetermined region is a region which is extensive in the widthwise direction W from one side edge part of the absorber 2 to the other side edge part.

Herein, in the pants-type diaper 1 according to the embodiment, such compression lines 4A are configured so as to be formed all over the regions of the absorber 2 including such a predetermined region.

Such compression lines 4A may be formed by means of emboss processing from the skin contact surface side of the pants-type diaper 1; may be formed by means of emboss processing from the cloth contact surface side of the pants-type diaper 1; or alternatively, may be formed by emboss processing from both surface sides.

In addition, such emboss processing may be directly applied to a core; may be applied from the top of a covering sheet 2C; or alternatively, may be applied from the top of the topsheet or the backsheet. Herein, emboss processing is applied from the top of the covering sheet 2C or the topsheet, thereby making it possible to avoid troubles of winding around a roll for applying emboss processing.

In a case of applying such emboss processing, it is desirable that a pressure to be applied to the roll for applying emboss processing is within a range from 10 MPa to 50 MPa; it is desirable that a temperature is within a range of 60 degrees Celsius to 100 degrees Celsius; and it is desirable that a line speed is within a range from 50 m/min to 500 m/min.

As shown in Fig. 5 (a) and Fig. 5 (b), in the absorber 2, a region 4B which is surrounded by the compression lines 4A which are adjacent to each other may be configured so as to be formed in the shape of rhombus (in the shape of a lattice).

Herein, as shown in Fig. 5 (a) and Fig. 5 (b), a dimension of the rhombus shape 4B in the widthwise direction W may be configured so as to be longer than that of the rhombus shape 4B in the longitudinal direction L.

That is, a distance Beta in the longitudinal direction L between rhombus-shaped apexes 4C which are adjacent to each other, is configured so as to be longer than a distance Alfa in the widthwise direction W between the rhombus-shaped apexes 4C which are adjacent to each other.

Specifically, as shown in Fig. 5 (b), it is desirable that the distance Alfa in the widthwise direction W between the rhombus-shaped apexes 4C which are adjacent to each other is within a range from 10 mm to 40 mm (for example, 20 mm); and it is desirable that a distance Beta in the longitudinal direction L between the rhombus-shaped apexes 4 which are adjacent to each other is within a range from 10 mm to 40 mm (for example, 25 mm).

In the absorber 2, the region 4B that is surrounded by the compression lines 4A which are adjacent to each other may be configured so as to be formed in a shape other than the rhombus shape.

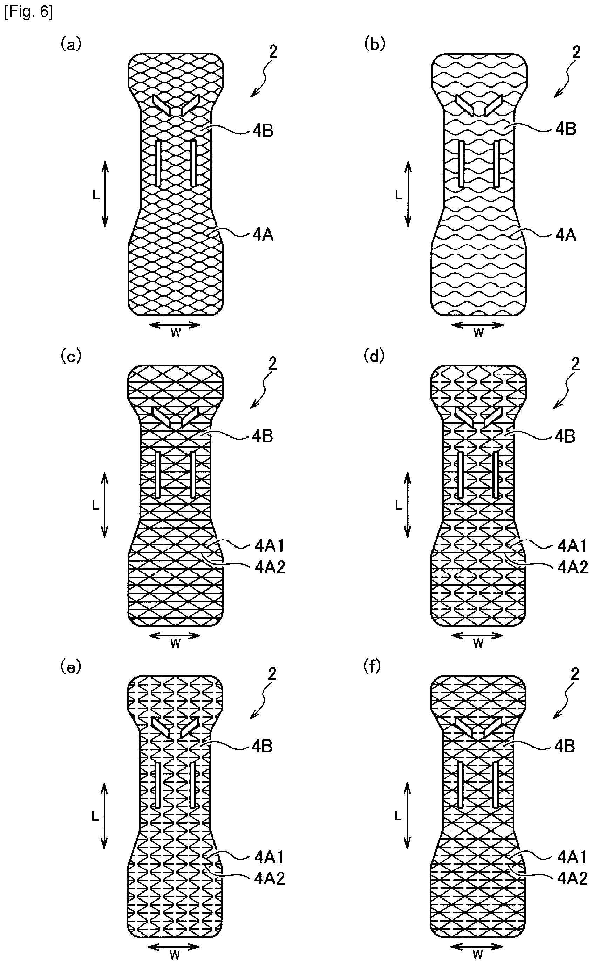

The region 4B that is surrounded by the compression lines 4A which are adjacent to each other , as shown in Fig. 6 (a) and Fig. 6 (b), may be formed in the shape that is surrounded by wavy compression lines 4A, or alternatively, this region, as shown in Figs. 6 (c) to (f), may be formed in the shape that is surrounded by a compression lines 4A1 which are inclined with respect to the widthwise direction W and a compression line 4A2 which is substantially parallel to the widthwise direction W.

In addition, the two compression lines 4A which are adjacent to each other in the longitudinal direction L are configured so as to approach each other at predetermined portions.

As shown in Fig. 5 (a) and Fig. 5 (b), in the absorber 2, in a case where the region 4B that is surrounded by the compression lines 4A which are adjacent to each other is configured so as to be formed in a rhombus shape, the two compression lines 4A which are adjacent to each other may be configured so as to cross at the apexes that are formed in the rhombus shape.

According to such a structure, since the two compression lines 4A which are adjacent to each other are linked with each other, the rigidity of the absorber 2 increases and a sore thigh in a case where an external force due to a wearer's movement is applied can be avoided.

In addition, in the longitudinal direction L, the two compression lines 4A which are adjacent to each other may be configured so as not to cross each other. According to such a structure, an advantageous effect that the covering sheet 2C is hardly broken even if it is tissue can be attained.

For example, as shown in Fig. 5 (b), it is desirable that the width of the groove of the compression line 4A is within a range from 0.5 mm to 5 mm (for example, 1mm), and it is desirable that the height of the groove of the compression line 4A is within a range from 0.1 to 8 mm (for example, 2 mm).

In addition, as shown in Fig. 5 (b), an angle formed by the centerline CL along the widthwise direction W located in the compression line 4A and the widthwise direction W is configured so as to be included within a range of 0 degrees to 45 degrees.

With the structure described above, the bending rigidity in the widthwise direction W in the pants-type diaper 1 is configured so as to be higher than that in the longitudinal direction L in the pants-type diaper 1.

The bending rigidity in the widthwise direction W in the pants-type diaper 1 and the rigidity in the longitudinal direction L in the pants-type diaper 1 can be measured by employing equipment called KES-FB as follows (relevant information can be retrieved from, e, g" "http://english.keskato.co.jp/products/").

In such equipment, two chucks called a movement chuck and a fixing chuck are provided, and the above-described rigidities can be measured by fixing samples to such two chucks.

Specifically, in such equipment, at the time of measurement, since the movements chuck moves on a circumference, with the fixing chuck being a start point, samples are bent, and by measuring a bending moment at that time, the sample bending rigidity (B [gf cm/cm]) can be measured.

In such equipment, the rigidity of bending in a region of 100 mm x 50 mm between the fixing chuck and the movement chuck can be measured. In order to compute an always uniform measurement result, two rhombus shapes are set so as to be included in such a region.

Specific measurement procedures are as follows.

First, a fixing chuck and a movement chuck are opened in accordance with a thickness of a sample, and the sample is inserted between the fixing chuck and the movement chuck; Second, a tightening screw of the movement chuck is secured; and third, a tightening screw of the fixing chuck is secured and then the sample is fixed.

Fourth, since the fixing chuck is linked with a torque meter by means of piano wire, a torque exerted by twisting of such a piano wire is detected.

Herein, there is shown a result obtained by performing the abovementioned measurement, employing a thin absorber sample formed in a square of 100 mm x 100 mm, of which a basis weight of pulp is 145 g/m², and a thick absorber sample of a square of 100 mm x 100 mm, of which a basis weight of pulp is 257 g/m².

First, the abovementioned measurement was performed for: the thin absorber sample to which emboss processing shown in Fig. 5 (a) and Fig. 5 (b) is applied; and the thin absorber sample to which emboss processing is not applied.

As a result, in the thin absorber sample to which emboss processing had been applied, the bending rigidity in the longitudinal direction L (MD direction) was obtained as "0.40B", and the bending rigidity in the widthwise direction W (CD direction) was obtained as "0.23B".

On the other hand, in the thin absorber sample to which emboss processing had not been applied, the bending rigidity in the longitudinal direction L (MD direction) was obtained as "0.22B", and the bending rigidity in the widthwise direction W (CD direction) was obtained as "0.21B".

Therefore, in the thin absorber sample to which emboss processing had been applied, it was found that the bending rigidity in the widthwise direction W is configured to be higher than that in the longitudinal direction L.

Second, the abovementioned measurement was performed for: the thick absorber sample to which emboss processing shown in Fig. 5 (a) and Fig. 5 (b) had been applied; and the thick absorber sample to which emboss processing had not been applied.

As a result, in the thick absorber sample to which emboss processing had been applied, the bending rigidity in the longitudinal direction L (MD direction) was obtained as "1.60B"; and the bending rigidity in the widthwise direction W (CD direction) was obtained as "1.18B".

On the other hand, in the thick absorber sample to which emboss processing had not been applied, the bending rigidity in the longitudinal direction L (MD direction) was obtained as "1.12B"; and the bending rigidity in the widthwise direction W (CD direction) was obtained as "1.10B".

Therefore, in the thick absorber sample to which emboss processing had been applied, it was found that the bending rigidity in the widthwise direction W is configured so as to be higher than that in the longitudinal direction L.

In this way, in the absorber to which emboss is applied, it is preferable that: the rigidity in the longitudinal direction L (MD direction) is 1.3 times or more of that in the widthwise direction W (CD direction); and that the bending rigidity in the widthwise direction W (CD direction) is within 1.1 times of that before emboss processing.

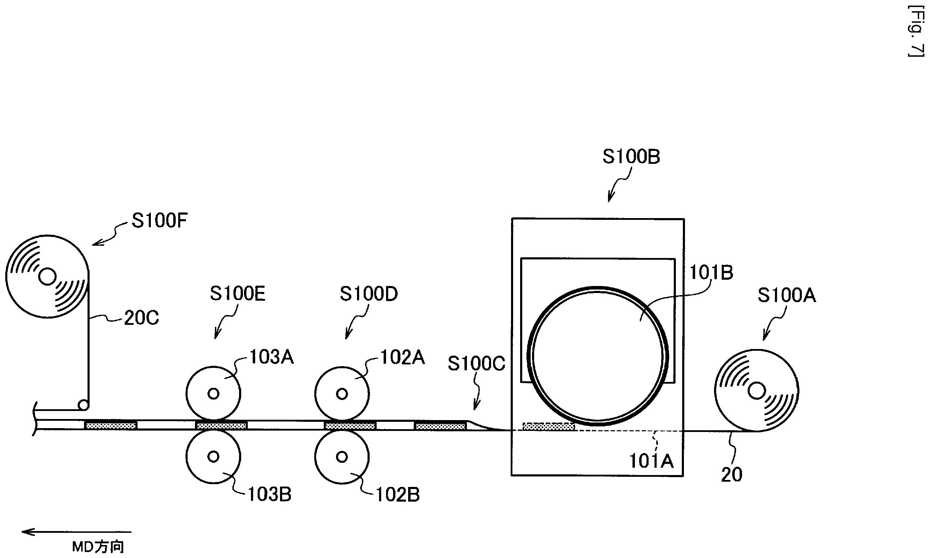

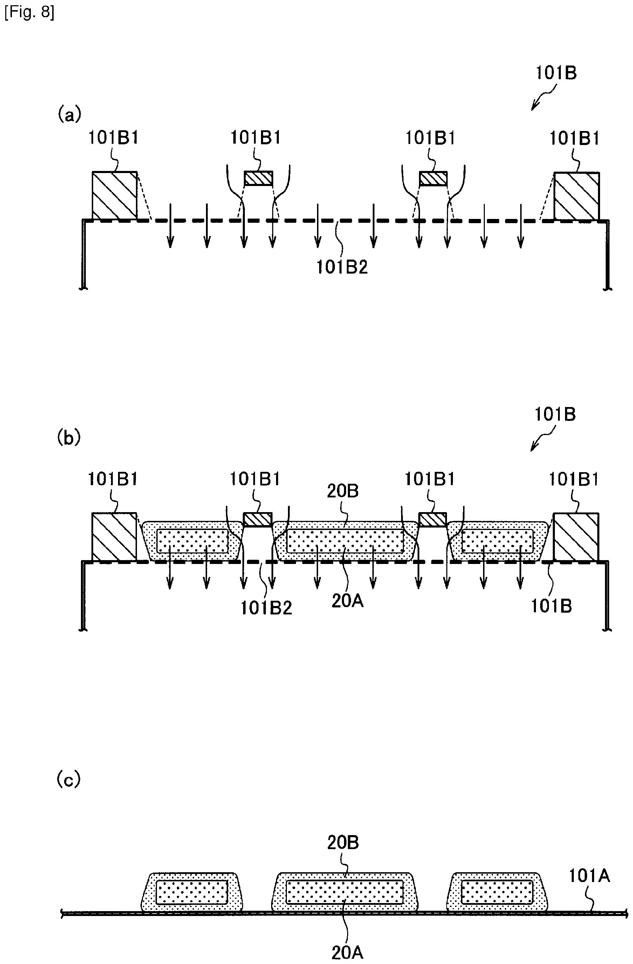

Hereinafter, part of a method of manufacturing the absorber 2 of the pants-type diaper 1, according to the embodiment, will be described with reference to Fig. 7 and Fig. 8. An existing method can be employed as the one which is not described in Fig. 7 and Fig. 8.

As shown in Fig. 7, in step S100A, a web for covering sheet (e.g., tissue) is conveyed in the MD direction by means of a conveyor belt 101A. Here, a hot melt adhesive for fixing the absorber 2 is applied onto the web for covering sheet.

In step S100B, a core made of pulp and SAP is integrated on an absorber lamination drum 101B.

Here, the absorber lamination drum 101B, as shown in Fig. 8 (a), is provided with a non-suction unit 101B1 and a suction unit 101B2.

Therefore, as shown in Fig. 8 (b), in step S100B, a slit 3A is formed by suctioning the core that is integrated on the absorber lamination drum 101B, via a section hole which is provided at the suction unit 101B2, and as shown in Fig. 8 (c), the core at which the slit 3A is formed is transferred onto the conveyor belt 101A.

In step S100C, the core is covered with the web for covering sheet by means of sailor.

In step S100D, the core is brought into pressure contact from a top of the web for covering sheet by means of flat press rollers 102A/102B, whereby bulkiness of the absorber 2 is restrained in advance. As a result, a breakage of the web for covering sheet can be prevented.

In step S100E, the core is brought into pressure contact from the skin contact surface side or the cloth contact surface side by means of the roller 103A/103B. Here, a pattern for emboss processing, shown in Fig. 5 (a) and Fig. 5 (b), is provided at the roller 103A.

In a case where emboss processing is performed from the skin contact surface side, a recessed part exists at the skin contact surface side of the absorber 2, thereby making it possible to reduce an area in which a surface of a topsheet wetted after discharging urine comes into contact with a wearer's skin.

In addition, in a case where emboss processing is performed from the cloth contact surface side, since a face to which emboss processing is applied exists at the backsheet side, and a covering sheet 2C and a film for backsheet are adhesively fully bonded with each other, SAP particles never moves arbitrarily from a broken portion; and therefore, as a result, a risk of leaking onto the surface of pants-type diaper 1 can be minimized.

In step S100F, a web for topsheet is attached to a core from the top of the web for covering sheet.

Since the absorbent article 1 is configured so as to form a compression unit 4A together with a folding lines 3A/3B, even in a case where the width of the absorber 2 disposed in an crotch region A is large, a side part of the absorber 2 can be easily lifted without degrading the softness in the longitudinal direction L. Therefore, a wearer's sense of wearing the article can be enhanced while urine leakage is avoided.

In addition, with the absorbent article 1 according to the embodiment, since compression lines 4A are formed in a predetermined region of the absorber 2 that is disposed in the crotch region A, and the compression lines 4A which are adjacent to each other in the longitudinal direction L is configured to approach in a predetermined site, the urine that is excreted by a wearer can be diffused in the longitudinal direction L, and a side leakage can be reduced.

### (Exemplary Modification)

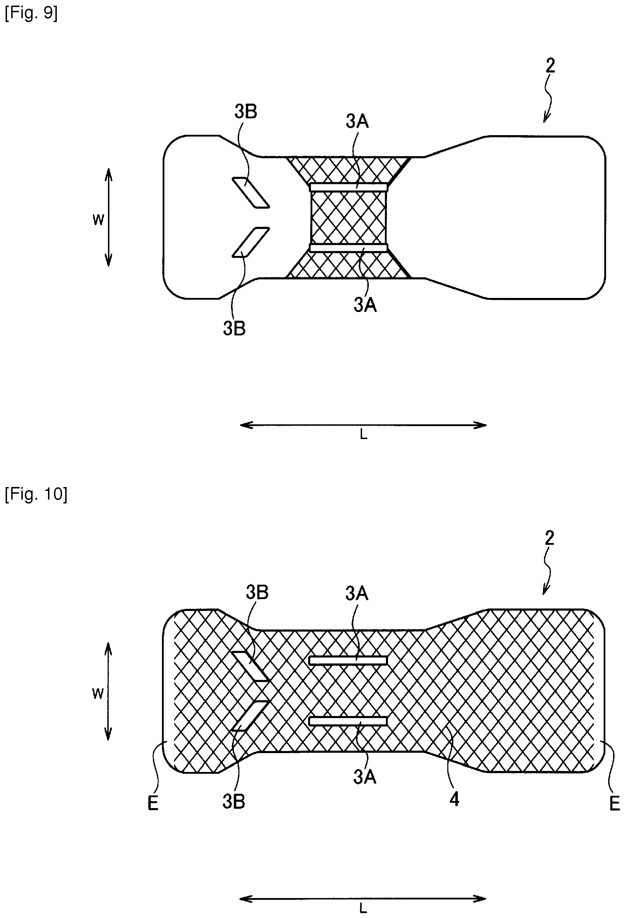

A pants-type diaper 1 according to Exemplary Modification of the present invention will be described with reference to Fig. 9 and Fig. 10. Herein, the pants-type diaper 1 according to the Exemplary Modification will be described focusing on differences from the pants-type diaper 1 according to the first embodiment.

As shown in Fig. 9 and Fig. 10, in the pants-type diaper 1 according to the Exemplary Modification, the compression line 4A is not formed in a full region of the absorber 2.

For example, as shown in Fig. 9, in a center region in the widthwise direction W of the inner-leg region A, the compression lines 4A are formed in a region which is extensive in the widthwise direction W from one side edge part of the absorber 2 to the other side edge part and in a region which is extensive in a region in which a folding line 3A is formed in the longitudinal direction L; and in a region between the center region in the widthwise direction W of the inner-leg region A and each of the side edge parts of the absorber 2, the compression line may be formed in a trapezoidal region which is extensive from the center region in the widthwise direction W of the inner-leg region A toward each of the side edge parts of the absorber 2.

In addition, the compression line 4A, as shown in Fig. 10, is not formed in a region B which is proximal to an end part in the longitudinal direction L, and may be formed all over other regions.

According to such a structure, since the compression line 4A is not formed in the region E that is proximal to the end part in the longitudinal direction L, the urine that is excreted and diffused by a wearer stays in such a region E, thereby making it possible to avoid a circumstance that the urine blurs out from the end part in the longitudinal direction L.

Hereinafter, while the present invention was described in detail by way of the above-mentioned embodiment, it is apparent to one skilled in the art that the present invention is not limited to the embodiments described in the present specification. The present invention can be carried out as aspects of alteration and modification without deviating from the gist and scope of the present invention defined by the recitations of the claims. Therefore, the description of the present specification is intended for illustrative explanation, and does not have any limitative meaning to the present invention.

For example, the present invention may be defined as follows. The compression lines may be formed in the pulp layer. Further folding lines inclined with respect to said folding lines and said further folding lines may be inclined at an identical inclination angle of the compression lines with respect to said folding lines.

Further the compression lines may be wavy lines, or the compression lines may not cross each other. Further the compression lines may include compression lines that are inclined with respect to the widthwise direction and compression lines that are substantially parallel to the widthwise direction.

## Claims

1. A pants-type diaper comprising:
an absorber (2) having a core including a pulp, the absorber being configured so that each face of the core is sandwiched by a covering sheet, wherein:
at least two folding lines (3A, 3B) which are extensive in a longitudinal direction of the pants-type diaper and which are spaced in a widthwise direction of the pants-type diaper (1) are formed at the absorber that is disposed in a crotch region of the pants-type diaper;
a plurality of compression lines (4A) which are extensive in a widthwise direction of the pants-type diaper (1) and which are spaced in the longitudinal direction of the pants-type diaper (1) are formed in a predetermined region of the absorber (2);
the predetermined region is a region which includes the folding lines (3A, 3B) and which is extensive in a widthwise direction from one side edge part to the other side edge part of the absorber (2);
an angle which is formed by a centerline along a widthwise direction located in the compression lines (4A) and the widthwise direction of the pants-type diaper (1) is configured so as to be included in a range of 0 degrees to 45 degrees;
and
two of the compression lines (4A) which are adjacent to each other in the longitudinal direction of the pants-type diaper (1) are configured so as to approach at a predetermined portion;
the core is made of: an intermediate layer (2A), in which a pulp and an SAP are mixed with each other, and a pulp layer (2B);
the folding line (3A) is formed by a slit; and
the pulp layer (2B) is configured so as to exist on a wall face of the slit and the intermediate layer (2A) is configured so as not to exist on the wall face of the slit; and the intermediate layer (2A) is configured so as to be disposed inside of the pulp layer (2B).

2. The pants-type diaper according to claim 1, wherein:
a region which is surrounded by the compression lines (4A) which are adjacent to each other is configured so as to be formed in a rhombus shape in the absorber (2); and
two of the compression lines (4A) that are adjacent to each other are configured so as to cross at an apex of the rhombus shape.

3. The pants-type diaper according to claim 2, wherein:
a dimension of the rhombus shape in a widthwise direction of the pants-type diaper (1) is configured so as to be larger than a dimension of the rhombus shape in the longitudinal direction of the pants-type diaper (1).

4. The pants-type diaper according to any one of claims 1 to 3, wherein:
the compression lines (4A) are not provided in proximity to an end part in the longitudinal direction of the pants-type diaper (1).

5. The pants-type diaper according to any one of claims 1 to 4, wherein:
a rigidity in the widthwise direction of the pants-type diaper (1) is configured so as to be higher than a rigidity in the longitudinal direction of the pants-type diaper (1).

## Patentansprüche

1. Höschenwindel umfassend:
einen Absorber (2), der einen Kern mit einem Zellstoff aufweist, wobei der Absorber so ausgebildet ist, dass jede Fläche des Kerns zwischen einer Abdecklage liegt, wobei:
zumindest zwei Faltlinien (3A, 3B), die sich in eine Längsrichtung der Höschenwindel erstrecken und die in eine Breitenrichtung der Höschenwindel (1) beabstandet sind, an dem in einer Schrittregion der Höschenwindel angeordneten Absorber gebildet sind;
eine Vielzahl an Kompressionslinien (4A), die sich in eine Breitenrichtung der Höschenwindel (1) erstrecken und die in eine Längsrichtung der hosenartigen Windel (1) beabstandet sind, in einer vorbestimmten Region des Absorbers (2) gebildet sind;
die vorbestimmte Region eine Region ist, die die Faltlinien (3A, 3B) umfasst und die sich in eine Breitenrichtung von einem Seitenkantenteil zu dem anderen Seitenkantenteil des Absorbers (2) erstreckt;
ein Winkel, der durch eine in den Kompressionslinien (4A) angeordnete Mittellinie entlang einer Breitenrichtung und die Breitenrichtung der Höschenwindel (1) gebildet ist, so ausgebildet ist, dass er in einem Bereich von 0 Grad bis 45 Grad liegt;
und
zwei der Kompressionslinien (4A), die zueinander in der Längsrichtung der Höschenwindel (1) benachbart sind, so ausgebildet sind, dass sie sich in einem vorbestimmten Abschnitt annähern;
der Kern besteht aus: einer Zwischenschicht (2A), in der ein Zellstoff und ein SAP miteinander gemischt sind, und einer Zellstoffschicht (2B);
die Faltlinie (3A) ist durch einen Schlitz gebildet; und wobei
die Zellstoffschicht (2B) so ausgebildet ist, dass sie auf einer Wandfläche des Schlitzes existiert, und die Zwischenschicht (2A) so ausgebildet ist, dass sie auf der Wandfläche des Schlitzes nicht existiert; und die Zwischenschicht (2A) so ausgebildet ist, dass sie innerhalb der Zellstoffschicht (2B) angeordnet ist.

2. Höschenwindel nach Anspruch 1, wobei:
eine Region, die von den zueinander benachbarten Kompressionslinien (4A) umgeben ist, so ausgebildet ist, dass sie in einer Rautenform in dem Absorber (2) gebildet ist; und
zwei der Kompressionslinien (4A), die zueinander benachbart sind, so ausgebildet sind, dass sie sich an einem Scheitel der Rautenform kreuzen.

3. Höschenwindel nach Anspruch 2, wobei:
ein Maß der Rautenform in eine Breitenrichtung der Höschenwindel (1) so ausgebildet ist, dass es größer ist als ein Maß der Rautenform in die Längsrichtung der Höschenwindel (1).

4. Höschenwindel nach einem der Ansprüche 1 bis 3, wobei:
die Kompressionslinien (4A) nicht in der Nähe zu einem Endteil in der Längsrichtung der Höschenwindel (1) vorgesehen sind.

5. Höschenwindel nach einem der Ansprüche 1 bis 4, wobei:
eine Steifigkeit in der Breitenrichtung der Höschenwindel (1) so ausgebildet ist, dass sie höher ist, als eine Steifigkeit in der Längsrichtung der Höschenwindel (1).

## Revendications

1. Couche-culotte comprenant :
un matériau absorbant (2) comprenant une partie centrale comprenant une pâte à papier, le matériau absorbant étant configuré de sorte que chaque face de la partie centrale soit intercalée par une feuille de revêtement,
dans laquelle :
au moins deux lignes de pliage (3A, 3B) qui sont extensibles dans une direction longitudinale de la couche-culotte et qui sont espacées dans une direction de la largeur de la couche-culotte (1) sont formées au niveau du matériau absorbant qui est disposé dans une région d'entrejambe de la couche-culotte ;
une pluralité de lignes de compression (4A) qui sont extensibles dans une direction de la largeur de la couche-culotte (1) et qui sont espacées dans la direction longitudinale de la couche-culotte (1) est formée dans une région prédéterminée du matériau absorbant (2) ;
la région prédéterminée est une région qui comprend les lignes de pliage (3A, 3B) et qui est extensible dans une direction de la largeur d'une partie de bord latéral vers l'autre partie de bord latéral du matériau absorbant (2) ;
un angle qui est formé par une ligne centrale le long d'une direction de la largeur située dans les lignes de compression (4A) et la direction de la largeur de la couche-culotte (1) est configuré de manière à être compris dans une plage de 0 degré à 45 degrés ;
et
deux des lignes de compression (4A) qui sont adjacentes l'une à l'autre dans la direction longitudinale de la couche-culotte (1) sont configurées de manière à s'approcher au niveau d'une partie prédéterminée ;
la partie centrale est composée : d'une couche intermédiaire (2A), dans laquelle une pâte à papier et un SAP sont mélangés l'un avec l'autre, et d'une couche de pâte à papier (2B) ;
la ligne de pliage (3A) est formée par une fente ; et
la couche de pâte à papier (2B) est configurée de manière à exister sur une face de paroi de la fente et la couche intermédiaire (2A) est configurée de manière à ne pas exister sur la face de paroi de la fente ; et la couche intermédiaire (2A) est configurée de manière à être disposée à l'intérieur de la couche de pâte à papier (2B).

2. Couche-culotte selon la revendication 1, dans laquelle :
une région qui est entourée par les lignes de compression (4A) qui sont adjacentes les unes aux autres est configurée de manière à présenter la forme d'un losange dans le matériau absorbant (2) ; et
deux des lignes de compression (4A) qui sont adjacentes l'une à l'autre sont configurées de manière à se croiser au niveau d'un sommet de la forme de losange.

3. Couche-culotte selon la revendication 2, dans laquelle :
une dimension de la forme de losange dans une direction de la largeur de la couche-culotte (1) est configurée de manière à être supérieure à une dimension de la forme de losange dans la direction longitudinale de la couche-culotte (1).

4. Couche-culotte selon l'une quelconque des revendications 1 à 3, dans laquelle :
les lignes de compression (4A) ne sont pas disposées à proximité d'une partie d'extrémité dans la direction longitudinale de la couche-culotte (1).

5. Couche-culotte selon l'une quelconque des revendications 1 à 4, dans laquelle :
une rigidité dans la direction de la largeur de la couche-culotte (1) est configurée de manière à être supérieure à une rigidité dans la direction longitudinale de la couche-culotte (1).
